# EUROPEAN PATENT APPLICATION

(11) **EP 1 642 986 A2**
(43) Date of publication of application: **05.04.2006**
(21) Application number: 05256122.2
(22) Date of filing: 29.09.2005
(51) Int. Cl.: C12Q 1/68

(54) **Method for screening cells and method for detecting oral carcinoma cells**

(30) Priority: 29.09.2004 JP 2004284342
(71) Applicant: CANON KABUSHIKI KAISHA, Ohta-ku, Tokyo (JP)
(72) Inventor: Sasaki, Kohsuke, Ube-shi Yamaguchi-ken (JP); Yamamoto, Nobuko Canon Kabushiki Kaisha, Tokyo (JP)
(74) Representative: Beresford, Keith Denis Lewis

(57) **Abstract**

The present invention provides a method of detecting oral carcinoma cells with high accuracy, and a method of making possible early detection of oral carcinoma or discrimination between a precancerous lesion and an early cancer in diagnosis of oral carcinoma. The methods are achieved by screening cells for oral carcinoma or precancerous lesion by measuring a DNA copy number in whole chromosomes or a part thereof in a sample, wherein chromosomal regions for which said copy number is measured comprise at least one region selected from the group consisting of: a 22-23 region in the q arm of Chromosome 8, a 14-21 region in the p arm of Chromosome 3 and a 12-22 region in the p arm of Chromosome 5.

SEQUENCE LISTING

<110> CANON KABUSHIKI KAISHA

<120> METHOD FOR SCREENING CELLS AND METHOD FOR DETECTING ORAL CARCINOMA CELLS

<130> 2972230

<150> JP 2004-284342
<151> 2004-09-29

<160> 1

<170> PatentIn version 3.1

<210> 1
<211> 22
<212> DNA
<213> Artificial

<220>
<223> PCR primer

<220>
<221> misc_feature
<222> (11)..(16)
<223> any one of "a", "g", "t" or "c"

<400> 1
ccgactcgag nnnnnnatgt gg 22

## Description

### BACKGROUND OF THE INVENTION

### Field of the Invention

The present invention relates to a method for screening oral carcinoma cells. The present invention also relates to a method for discriminating between cells in precancerous condition (precancerous lesion cells) and oral carcinoma cells, and detecting oral carcinoma cells early.

### Related Background Art

Oral carcinoma is estimated to be the 6th most frequent cancer in the world and occurs at high frequency especially in certain areas of Asia. Oral carcinoma includes cancer of the maxillary sinus, which is located inside the left and right cheek, cancer of the tongue in the mouth, cancer of the epipharinx in the nose or in the deep throat, cancer of the larynx in the periphery of the vocal cord and the like. In spite of the fact that they are very common cancer, the prognosis is not good, and there are frequent recurrences and in many cases, death is the result. One of the reasons for these is the difficulty in early detection. In many cases, there are very few subjective symptoms until the cancer progresses to an advanced stage and it may be already too late to excise the tumor due to metastasis and infiltration when the definite diagnosis is made from the symptoms of the ear and nose, and the pain in the tongue. Thus, early detection is desired earnestly and it is important to know the degree of malignancy of the tumor to give an effective treatment.

The diagnostic method used as of now is based on the pathological method for the subject area, and experienced pathologists examine the stained tissues under a microscope to give diagnosis.

However, the pathological diagnosis in oral carcinoma is sometimes difficult due to the poor quality of biopsy samples. Furthermore, there is a wide variety in the diagnostic capability and the experience of the physician in charge and in the performance of the testing equipment such as the microscope and the like, and these make the early detection difficult.

In the diagnostic method using tumor markers, the degree of expression of the tumor markers EGFR and Her2 typically used are poorly correlated with the progression of oral carcinoma. At this time no other genetic marker is known for predicting the presence of a cancer, and it has been impossible to predict and make early diagnosis for oral carcinoma by detecting tumor markers.

A method is proposed to solve the problem mentioned above by using an abnormality of the copy number of chromosomal DNA as a marker for cancer diagnosis. The CGH method is known as a method for detecting an increase or decrease of the copy number of chromosomal DNA (for example, refer Japanese Patent Application Laid-Open No. H07-505053). In this method, DNAs are extracted from normal and tumor tissues, and the labeled normal DNA and tumor DNA are subjected to competitive in situ hybridization on metaphase chromosomes using a DNA probe directly labeled with fluorescent dye. The resultant images are captured by a CCD camera, and fluorescent intensity ratio of tumor/normal is measured. DNAs of tumor cells and normal cells are labeled with different dyes, and if there are an equal number of tumor and normal cells, the ratio is 1 (or always a constant value). In a chromosome region where the ratio is high, it is judged that there is an increase in the copy number of tumor cells, that is, an amplification of the chromosomal region, and in a chromosomal region where the ratio is low, there is a decrease of the copy number, that is, the loss of the chromosomal region.

Weber et al. in Germany carried out the CGH on small samples and paraffin embedded materials for analyzing abnormality in DNA copy number in biopsy samples (pathological examination before operation) and reported, although the number of cases was small, accurate data by collecting tumor cells with high purity by the microdissection method from lesions of infiltrated cancer, epithelial cancer and epithelial dysplasia from the same patient.

In oral carcinoma, some investigations have been carried out using samples from a few cases and cell lines (for example, "Applied Cytometry" edited by Yoshio Tenjin, Igaku Shoin). Some regions with high correlation to oral carcinoma have been pointed out, but at the moment it is not the situation where every investigator agreed on, and there has been no report analyzing the correlation with the progression of cancer in a large number of cases. Thus, there is neither the identification of the specific chromosomal regions required for screening, nor the disclosure of the chromosomal region which makes possible the discrimination of precancerous lesion from cancer.

### SUMMARY OF THE INVENTION

As described above, attempts have been made to analyze abnormality in the DNA copy number for cancer diagnosis, but no method for early diagnosis for oral carcinoma is found yet, because the chromosomal region where abnormality in the DNA copy number occurs in oral carcinoma cells is not yet found. Also, at this time it is almost impossible to evaluate the degree of malignancy of the cancer before starting the treatment.

The present inventors have analyzed chromosomal DNA of oral carcinoma patients and of normal healthy people by the CGH method described above and have succeeded to extract a region where chromosomal DNA increases or decreases with a high correlation with oral carcinoma. Further, they have discovered that the patients with oral carcinoma can be distinguished with high accuracy by comparing the amount of chromosomal DNA in the test sample with that of normal healthy people. They have also performed correlation analyses between the increase or decrease in the DNA copy number of these regions and the results of pathological analysis of the tissues of patients, and have invented a method for predicting the degree of progression of cancer based on the increase or decrease of the copy number of the chromosomal DNA which has a strong correlation with the degree of progression of cancer and the lymph node metastasis.

Thus, the present invention demonstrates the chromosomal regions, which are useful for early detection of oral carcinoma based on the increase or decrease of the copy number of DNA specific to oral carcinoma patients. The present invention also demonstrates the chromosomal regions which are useful for discriminating precancerous lesion from early cancer and the chromosomal regions which are useful for evaluating the degree of malignancy and as targets for anti-cancer drug development and chemical prevention. An embodiment of the present invention provides a method for screening oral carcinoma cells or their precancerous lesion cells using the chromosomal regions described above. Another embodiment provides a DNA array in which clones of the chromosomal regions which are useful for detecting oral carcinoma cells or their precancerous lesion cells are fixed on a substrate.

A method according to an emnbodiment of the present invention screens cells for oral carcinoma or precancerous lesion by measuring a DNA copy number in whole chromosomes or a part thereof in a sample in vitro, wherein chromosomal regions for which the copy number described above is measured, includes at least one region selected from the group consisting of a 22-23 region in the q arm of Chromosome 8, a 14-21 region in the p arm of Chromosome 3 and a 12-22 region in p arm of Chromosome 5.

The screening described above is preferably carried out by comparing the measured copy number with a chromosomal DNA copy number of normal healthy people:

It is preferable that the chromosomal regions where the copy number is measured further include at least one of the 23 autosomal regions described herein in Table 1.

It is more preferable that the chromosomal regions include at least one of the 7 chromosomal regions described herein in Table 2.

It is preferable to detect an increase in the copy number of the 22-23 region in the q arm of Chromosome 8.

Another embodiment of the present invention provides a method for detecting oral carcinoma cells.

A first method of the embodiment for detecting oral carcinoma cells measures a chromosomal DNA copy number, wherein the region 14-21 of the p arm of Chromosome 3 is detected having a lower copy number than in normal healthy people.

A second method of the embodiment for detecting oral carcinoma cells measures a chromosomal DNA copy number, wherein the region 12-22 of the p arm of Chromosome 5 is detected having a lower copy number than in normal healthy people.

And another embodiment of the present invention also provides an array device having the clones of the chromosomes described above fixed on a substrate thereof.

The clones fixed on the substrate are preferably bound to a labeled DNA of normal healthy people by a hybridization reaction.

The present invention makes detection of oral carcinoma cells possible with high accuracy. In the diagnosis of oral carcinoma, the present invention also makes possible early detection or discrimination between precancerous lesion and early cancer and, furthermore, provides an effective method for evaluating the degree of malignancy of cancer.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

FIG. 1 shows regions with abnormal DNA copy number in squamous cell carcinoma and epithelial dysplasia; and

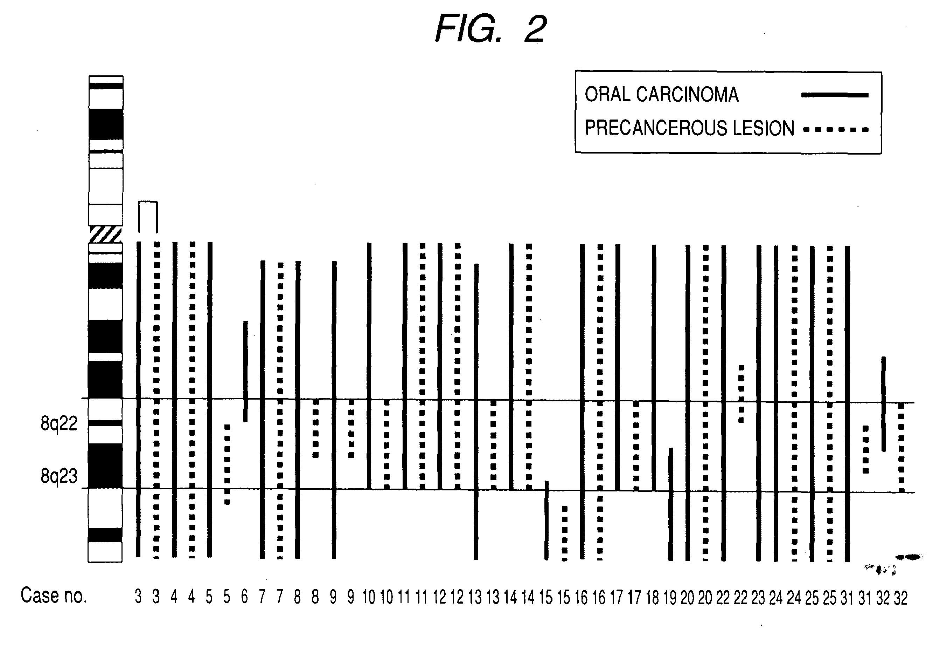

FIG. 2 shows increase in copy number in the 8q22 region in oral carcinoma and precancerous lesions.

### DETAILED DESCRIPTION OF THE PREFERRED EMBODIMENTS

Preferred embodiments of the present invention will now be described in detail in accordance with the accompanying drawings, and it is to be understood that any of the embodiments is an example to appropriately practice the present invention and in no way limits other embodiments according to the present invention.

The present invention provides, as described below, a method for cancer diagnosis by identifying the region where the chromosomal abnormality occurs in oral carcinoma cells and by detecting abnormality of the copy number of the specific region.

Thus the specific region will now be explained in detail and the methods using this region will be described later.

### (1) Identification of the region with abnormality in DNA copy number in oral squamous cell carcinoma (OSCC) and dysplasia (DYS).

Samples of cancer tissues and juxtaposing precancerous dysplasia lesion were collected by biopsy from 35 patients with oral carcinoma. Among the cancer patients, 13 patients had a mild carcinoma, 11 moderate and 11 severe. The age of the patients ranged from 48 to 91 years old. Eighteen patients were males and 17 were females. Samples were used for investigation after obtaining informed-consent from all the patients.

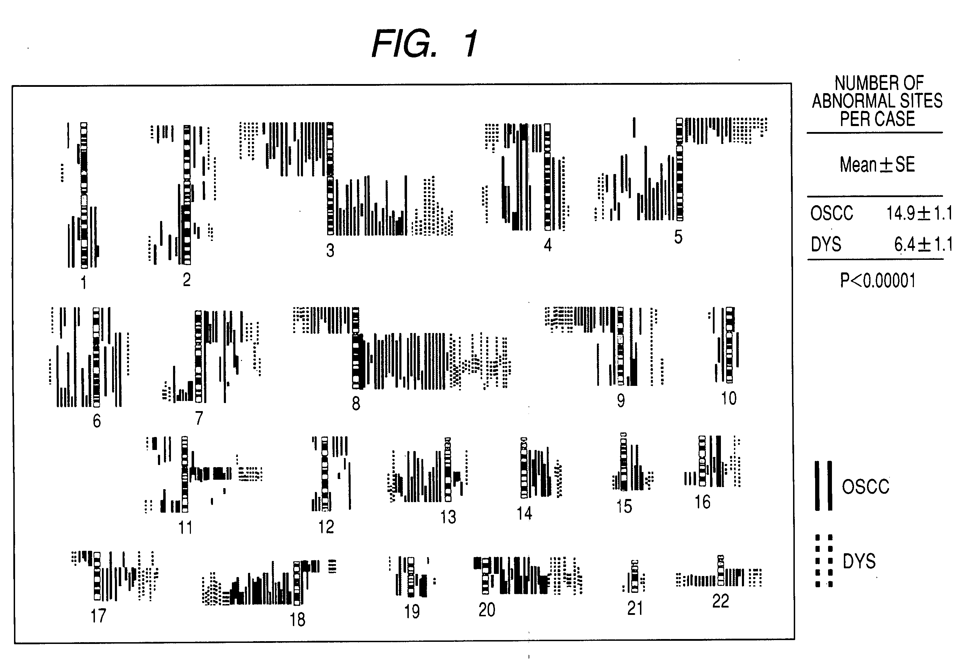

DNAs were extracted from these samples. DNAs from cancer were labeled with SpectrumGreen-dUTP (Vysis Inc.) and DNA extracted from lymphocytes of normal healthy people was labeled with SpectrumRed-dUTP (Vysis Inc.) by the nick translation method, and increase or decrease of chromosomal DNA was compared with the CGH method. FIG. 1 shows chromosomal regions where the correlation is observed. P in FIG. 1 represents the correlation function. When the ratio of DNA copy number in whole chromosomes or a part thereof in the sample to the copy number in normal healthy people is 1.2 or above, it is defined as amplification, and when the ratio was 0.8 or less, it is defined as loss. The results were mapped on the chromosomal map. Locations where the DNA copy number is amplified are shown in the right side of the chromosomes and where the DNA copy number is lost are shown in the left side of the chromosomes. The solid lines represent oral carcinoma and the broken lines represent precancerous lesion. Table 1 shows the chromosomal regions with a marked change in the copy number, classified according to the histopathological degree of malignancy of the precancerous lesion. The DNA copy number is either increased or decreased in 23 locations of chromosomal regions relative to that in normal healthy people.

In precancerous mild lesion, the DNA copy number is almost the same as in normal healthy people in 22 chromosomal regions out of 23 chromosomal regions in Table 1. The change in the DNA copy number occurs with aggravation of the lesion, and in oral carcinoma, from 30% to 80% of the total samples demonstrate either increase or decrease of the DNA copy number in each region.

Therefore, it is possible to screen oral carcinoma by using the 23 locations described in Table 1 as a marker. Among the 23 locations, the 7 locations (Table 2), in which the abnormality in the copy number is observed in more than half of the oral carcinoma patients, may be used for screening with higher accuracy.

As seen in FIG. 1, the increase in the copy number in the q arm of Chromosome 8 is observed in most of the samples. Actually, as shown in Table 1, the copy number of the 8q22-23 region is increased even in about half of the precancerous mild lesions. Detailed examination of amplified locations reveals the increase of the copy number in the 8q22 region both in oral cancer and in precancerous lesion. That is, the copy number in the 8q22-23 region is increased by 46% in mild samples, by 90% or above in moderate samples and by 80% or more in severe samples.

Therefore, it can be said that oral cancer patients or people prone to have cancer (with precancerous lesion) may be screened easily by paying attention to the copy number of this location and by detecting the increased copy number in 8q22 region relative to that of normal healthy people.

Next, according to Table 1, when the lesion progresses from mild to moderate, the amplification is observed in 3q26-qter, 8q11-q21, 8q24.1-qter, 20q and the decrease of the copy number is seen in 18q22-qter and 3q14-21.

When the lesion progresses from moderate to severe, the amplification is observed in 11q13, 14q, 17q11-22 and 20q, while the loss is observed in 9p.

The 23 locations of the chromosomal regions shown in Table 1 may be further used for screening oral cancer as useful chromosomal regions.

Comparison of the tissues of precancerous lesions and the tissue diagnosed as oral carcinoma has revealed marked loss in 3p14-21 and 5q12-22. The progression from precancerous condition to oral carcinoma may be diagnosed by investigating the change in the DNA copy number in these chromosomal regions.

Since the 23 locations in Table 1 are identified as the chromosomal regions where the copy numbers are abnormal, by investigating the region in more detail and identifying the gene, it may be possible to contribute in developing anticancer agents which suppress the amplification/loss of the gene.

That is, these are the useful chromosomal regions as a target for development of anticancer agents/chemical prevention.

**(Table 1)**

| Location of copy number change | | Pre-cancerous mild lesion (%) | Pre-cancerous moderate lesion (%) | Pre-cancerous severe lesion (%) | Oral carcinoma (%) |
|---|---|---|---|---|---|
| 3q14-21 | loss | 0 | 18.2 | 9.1 | 57.1 |
| 3q26-qter | amplification | 0 | 54.5 | 63.6 | 74.2 |
| 4p | loss | 18.2 | 0 18.2 | 36.4 | 40.0 |
| 4q | loss | 0 | 0 | 18.2 | 34.3 |
| 5p15 | amplification | 0 | 54.5 | 54.5 | 45.7 |
| 5q12-22 | loss | 0 | 9.1 | 9.1 | 48.6 |
| 5q31-qter | loss | 0 | 9.1 | 27.2 | 54.2 |
| 6q | loss | 0 | 0 | 18.2 | 34.3 |
| 7p | amplification | 0 | 18.2 | 18.2 | 42.9 |
| 8p | loss | 0 | 27.3 | 18.2 | 37.1 |
| 8q11-q21 | amplification | 7.7 | 45.5 | 63.6 | 80.0 |
| 8q22-q23 | amplification | 46.2 | 90.9 | 81.8 | 88.6 |
| 8q24.1-qter | amplification | 0 | 36.4 | 63.6 | 74.3 |
| 9p | loss | 0 | 18.2 | 81.8 | 45.7 |
| 11q13 | amplification | 7.7 | 0 | 72.7 | 48.6 |
| 13q11-21 | loss | 0 | 0 | 9.1 | 31.4 |
| 13q31-32 | loss | 0 | 9.1 | 27.3 | 51.4 |
| 14q | amplification | 0 | 0 | 36.4 | 37.1 |
| 17q11-22 | amplification | 0 | 0 | 54.5 | 37.1 |
| 18p | amplification | 0 | 18.2 | 18.2 | 31.4 |
| 18q22-qter | loss | 7.7 | 45.5 | 54.5 | 80.0 |
| 20p | amplification | 0 | 18.2 | 36.4 | 37.1 |
| 20q | amplification | 7.7 | 18.2 | 81.8 | 68.6 |

**(Table 2)**

| Location of copy number change | | Precancerous Precancerous mild lesion (%) | Oral Oral carcinoma (%) |
|---|---|---|---|
| 3q14-21 | loss | 0 | 57.1 |
| 3q26-qter | amplification | 0 | 74.2 |
| 8q11-q21 | amplification | 7.7 | 80.0 |
| 8q22-q23 | amplification | 46.2 | 88.6 |
| 8q24.1-qter | amplification | 0 | 74.3 |
| 18q22-qter | loss | 7.7 | 80.0 |
| 20q | amplification | 7.7 | 68.6 |

### (2) CGH method

The CGH (Comparative Genomic Hybridization) method, which has been known as a method for detecting increased and decreased copy numbers in a chromosomal DNA, is a superior method which allows the detection of increased and/or decreased gene copy numbers in a tumor DNA at the same time by a single hybridization and can map these regions on all the chromosomes (Kallioniemi, A. et al. Comparative genomic hybridization for molecular cytogenetic analysis of solid tumors. Science 258:818-821, 1992).

The present invention provides copy number change on a chromosomal DNA measured by the CGH method described above, and thereby provides a method for screening oral carcinoma or a method for detecting oral carcinoma cells for diagnosis of oral carcinoma based on the increase or decrease of the copy numbers in the chromosomal regions described in (1).

### (3) Clone chip

The present invention provides a method for detecting increase or decrease of chromosomal DNA more conveniently using so called DNA microarray, in which the genes such as BAC clones covering the chromosomal regions shown in (1) and the like are fixed on a substrate.

That is, the BAC clones containing the chromosomal regions, which are required for screening of oral carcinoma described above, are selected from the BAC clone library of whole region of the human genome prepared by the normal method. These BAC clones may be a single clone or multiple clones per each region.

The carrier, on which the BAC clones are fixed, may be a flat plate like a glass substrate or beads and the like. To make the location of each BAC clone clear, it is necessary, for example, for BAC clones to correspond to the address on the substrate.

The present invention will be explained based on the microarray fixed on the glass substrate but the embodiment of the invention is not limited to this type of microarray.

The glass plate is prepared beforehand so that BAC clones can be fixed, by treating with silane coupling agent and other agents needed for crosslinking. Each solution of the BAC clones is spotted by the pin method or ink-jet method on the treated glass substrate, reacted and fixed on the surface of the substrate. The ink-jet method is more preferable at this step because it allows the content of a BAC clone in each spot constant. Usually the scattering of the spotted amount is not a problem in the CGH method because the method is designed to use the competitive reaction between the sample genome and the genome of normal healthy people. However, if the amount of the BAC clone can be controlled at a constant level, good reproducibility of the evaluation result is expected, and this makes the evaluation only by the sample itself possible. That is, the result at the same level as CG method can be obtained by detecting signals of normal healthy people followed by detecting those of various samples, and calculating the ratio of signals of samples to that of normal healthy people. This is done by using the BAC clone of the chromosomal region in which the copy number is the same between oral carcinoma and normal healthy people as the internal standard and calculating the ratio of the sample to the standard. To simplify the competitive reaction with normal healthy people, it may also be possible that the genomic DNA of normal healthy people is labeled with a different dye from that used for sample and bound to microarray beforehand.

The probes of the present invention are not limited to BAC clones, but synthetic oligonucleotides may be used as probes, as long as the performance is equivalent.

### (4) Evaluation of chromosomal DNA copy number

One of the methods for evaluating increase or decrease of chromosomal DNA copy number is to judge based on the ratio of the copy number of normal healthy people to that of the sample. That is, when the sample obtained by microdissection is labeled with green fluorescence and normal healthy people is labeled with red, if the copy numbers are equal (normal), the fluorescent light obtained is a middle color of the two kinds of fluorescence, that is, yellow fluorescence. However, when there is an amplification of chromosomes in the sample, there is more green fluorescence and the evaluation result tends to be green. On the other hand, when there is a loss in the sample, the sum of both types of fluorescence becomes red.

The ratio to normal healthy people is calibrated to be 1 using the standard gene which copy number would be stable, and based on this the DNA copy number in each chromosomal region is evaluated. In *in* situ hybridization, the increase or decrease of the copy number of each chromosomal region is judged by the standard that the ratio of sample/normal healthy people 1.2 or above is amplification and 0.8 or below is loss. However, the present invention is not limited to these values and, in the case of microarray using BAC clones, these values have to be optimized in accordance with the production method.

Further, if the measurements with higher accuracy are possible, the judgment may be made from the relative ratio of the copy numbers between the different chromosomal regions in the sample cells.

The present invention will be explained in detail using embodiments as follows.

### <Example 1>

### Screening of cells by the CGH method

### 1. DNA extraction

To maximize the detection sensitivity of abnormality in the CGH method, samples were prepared by the microdissection method to collect cancer cells selectively.

In particular, biopsy samples were stored at -80°C, and tissue sections with 9 µm thickness were prepared. After staining with methyl green or hematoxylin-eosin, the tissue section was recovered as 15 µm spots by laser capture dissection and extracted with SepaGene Kit (Sanko Junyaku Co.) to obtain DNA.

### 2. DOP-PCR

DOP (degenerate oligonucleotide-primed)-PCR was carried out using a universal primer-6-MW (5'-CCGACTCGAGNNNNNNATGTGG-3') (SEQ ID NO. 1).

In particular, to 1 µl of DNA obtained by microdissection, 4 µl of sequenase buffer and 1 U of topoisomerase (Promega Co. Trade Mark, Madison, WI) were added and incubated at 37°C for 30 min. Next, the reaction mixture was mixed with 20 U of Thermosequenase (Amersham Co. Trade Mark, Cleveland, OH) and subjected to 5 cycles of 94°C for 1 min, 30°C for 2 min and 37°C for 2 min. After heating at 95°C for 10 min, 2.5 U of Taq polymerase (Takara Co., Trade Mark, Shiga) was added to 45 µl of the PCR solution and subjected to 35 PCR cycles of 94°C for 1 min, 56°C for 1 min and 72°C for 3 min. The control DNA obtained from lymphocytes (normal DNA) was also subjected to DOP-PCR.

### 3. Labeling of DNA by nick translation

Using CGH Nick translation kit (Vysis Co.), cancer DNA and normal DNA were labeled with Spectrum Green and Spectrum Red, respectively.

### 4. CGH method

### 4-1. Preparation of single stranded probe

1) Preparation of probe

| | |
|---|---|
| Human Cot-1DNA | 10 µl |
| Lymphocyte DNA from normal healthy people (labeled with Spectrum Red) | 10 µl |
| Tumor DNA (labeled with Spectrum Green) | 20 µl |
| 3M sodium acetate | 40 µl |
| 100% ethanol | 110 µl |

The above reagents were added sequentially from the top, mixed well by inversion while shielded from light, kept at -80°C at least for 20 min and then centrifuged at 14,000 rpm at 4°C for 30 min.
2) The supernatant was discarded, the residual liquid was removed by suction and the precipitates were dried well while shielded from light (for about 1 hour).
3) Ten µl of master mix (dextran sulfate 1 g, 20 x SSC 1 ml, formamide 5 ml) was added and pipetted well while avoiding bubble formation. Liquid attached to the wall of the tube was spun down and the reaction tube was immersed in a 37°C waterbath.

### 4-2. Denaturation of DNA of chromosomal samples to make them single-strand

1) Chromosomal samples (on a slide glass) were treated with 70% formamide/2 x SSC at 72°C for 25 min to denature double stranded DNA to single stranded DNA.
2) Samples were dried by immersing in 70, 85 and 100 % ethanol for 2 min each.

### 4-3. Hybridization reaction

1) Ten µl of the probe mix prepared in 4-1 was added to a slide glass of the chromosome samples prepared in 4-2. After being covered with a 18 mm x 18 mm cover glass, the slide glass was placed on a 37°C hot plate for 10 min and then incubated at 37°C in a CO₂ incubator or 3 to 4 days for hybridization reaction.
2) The slide glass was washed with 50% formamide/2 x SSC at 45°C for 7 min. The washing was repeated 2 more times.
3) The slide glass was washed with 2 x SSC at 45°C for 7 min, with PN buffer at room temperature for 5 min and with distilled water at room temperature and dried.
4) The slide glass was treated with 8 µl of 0.1-0.2 mg/ml DAPI-II, sealed with a cover glass and observed.

### 4-4. Detection

1) Images were obtained with an Olympus BX650 fluorescent microscope with a CCD camera and analyzed with digital image analysis system (QUIPSTMXL, Vysis Co.).
2) The copy numbers of cancer and normal DNA were compared, and the ratio of 1.2 or above was judged to be amplification and 0.8 or below was judged to be loss. When the ratio was over 1.4, it was judged that the copy number was markedly increased.

### 4-5. Analysis

1) The change in the copy number and the degree of progression of cancer of the patient were subjected to correlation analysis to identify the gene involved.
2) FIG. 1 and Table 1 show the chromosomal regions where amplification or loss of the copy number according to the index of 4-4 was observed.

The result indicated clearly that the screening for oral carcinoma cells can be carried out by analyzing the amplification or loss of the copy number in each region which was discovered by the present invention as described above.

### <Example 2> (Preparation of BAC clone microarrays)

Slide glasses were washed and treated with aminosilane coupling agent according to the conventional method.

The clones corresponding to the chromosomal regions, which were determined to be useful for screening of oral carcinoma based on the result of the analysis in Example 1 (Table 1), were selected from the BAC clone library prepared by the conventional method. These clone solutions were injected into a 96 well microtiter plate and spotted on the slide glass with a DNA microarray apparatus. After standing at room temperature and fixing, the microarray was subjected to competitive hybridization reaction with DNAs of normal healthy people and samples labeled with different fluorescent dyes to obtain the ratio of copy numbers of DNAs of samples and normal healthy people. By defining the ratio of 1.2 or above as amplification and 0.8 or below as loss, the results obtained were almost the same as Example 1.

### <Example 3> (Preparation of the back clone microarray with DNA of normal healthy people fixed beforehand)

A back clone microarray was prepared as described in Example 2, and DNA of lymphocytes of normal healthy people, which was labeled with Spectrum Red, was fixed thereto by hybridization.

Sample DNA was labeled with Spectrum Green and hybridized with the microarray in which DNA of normal healthy people was already bound to back clones.

The red fluorescence of DNA of normal healthy people, which was originally bound to back clones, was replaced with the green fluorescence derived from the sample, and the ratios between the two were similar to those in Example 2.

### <Example 4>

A microarray was prepared in a similar manner to Example 2 except that the concentration of each back clone was adjusted so that the copy number of back clones was equal. The microarray was reacted with labeled samples, and the result was analyzed. In this reaction, DNA of normal healthy people was not added, and the competitive hybridization was not carried out.

Five samples of oral carcinoma patients were subjected to the experiment and the fluorescence was compared. There were regions in 5 samples which demonstrated almost the same degree of fluorescence at the clones of the p arm of Chromosome 1 and the q arm of Chromosome 11. In FIG. 1, these regions correspond to the regions where neither amplification nor loss is observed (for example, the q arm of Chromosome 1 and the p arm of Chromosome 11). The fluorescence per unit length was calculated as an index using the fluorescence of these regions as a control, and the fluorescence ratio of each chromosomal region to the index was calculated to judge whether there was amplification or loss.

The result indicated that the increase in the copy number in the 22-23 region in the q arm of Chromosome 8 was detected in all the samples.

The present invention is not limited to the above embodiments and various changes and modifications can be made within the scope of the present invention. Therefore to apprise the public of the scope of the present invention, the following claims are made.

## Claims

1. A method for screening cells for oral carcinoma or precancerous lesion by measuring a DNA copy number in whole chromosomes or a part thereof in a sample,
wherein chromosomal regions for which said copy number is measured comprise at least one region selected from the group consisting of: a 22-23 region in the q arm of Chromosome 8, a 14-21 region in the p arm of Chromosome 3 and a 12-22 region in the p arm of Chromosome 5.

2. A method for screening cells according to claim 1, wherein the cell screening is carried out by comparing said measured copy number with a chromosomal DNA copy number of normal healthy people.

3. A method for screening cells according to claim 1, wherein the chromosomal regions in which said copy number is measured further include at least one of the 23 autosomal regions described herein in Table 1.

4. A method for screening cells according to claim 3, wherein the chromosomal regions in which said copy number is measured further include at least one of the 7 autosomal regions described herein in Table 2.

5. A method for screening cells for oral carcinoma according to claim 1, wherein an increase in the copy number of the 22-23 region in the q arm of Chromosome 8 is detected.

6. A method for detecting oral carcinoma cells, wherein a chromosomal DNA copy number is measured and the region 14-21 of the p arm of Chromosome 3 having a lower copy number than in normal healthy people is detected.

7. A method for detecting oral carcinoma cells, wherein a chromosomal DNA copy number is measured and the region 12-22 of the q arm of Chromosome 5 having a lower copy number than in normal healthy people is detected.

8. An array device having the clones of the chromosomes according to claims 1 to 7 fixed on a substrate thereof.

9. An array device according to claim 8, wherein a labeled DNA of normal healthy people is bound to said clones fixed.on said substrate by a hybridization reaction.
